# EUROPEAN PATENT APPLICATION

(11) **EP 3 009 841 A1**
(43) Date of publication of application: **20.04.2016**
(21) Application number: 14188831.3
(22) Date of filing: 14.10.2014
(51) Int. Cl.: G01N 33/566, G01N 33/68

(54) **Detection of IgM Antibodies using an immune complex (IC) ELISA**

(71) Applicant: Bernhard-Nocht-Institut für Tropenmedizin, 20359 Hamburg (DE)
(72) Inventor: Emmerich-Paloh, Petra, 20251 Hamburg (DE); Schmitz, Herbert, 22143 Hamburg (DE); Deschermeier, Christina, 22459 Hamburg (DE)
(74) Representative: Moré, Solveig Helga

(57) **Abstract**

The present invention relates to the field of diagnostic or analytic methods. In particular, the inventors teach that an Fc receptor for IgM heavy chains (FcµR), in particular, Faim 3/Toso or CD351, or a functional fragment thereof, which preferably comprises the Ig-like domain of Faim 3/Toso, may be used for *in vitro* quantification of IgM or IgA antibodies, preferably, IgM, in the form of immune complexes, i.e., complexes formed by antigen and specific antibody. These immune complexes may be formed by mixing an antigen with a sample comprising antibodies. A method for detection and quantification of IgA or IgM antibodies in the form of immune complexes is also provided. This can be useful for diagnosis of infections, e.g., with a virus.

SEQUENCE LISTING

<110> Bernhard-Nocht-Institut fuer Tropenmedizin

<120> Detection of IgM antibodies using an immune complex (IC) ELISA

<130> BNT15396EP

<160> 8

<170> BiSSAP 1.3

<210> 1
<211> 390
<212> PRT
<213> Homo sapiens

<220>
<221> SIGNAL
<222> 1..16

<220>
<223> HsFcÎ¼R

<220>
<221> TRANSMEM
<222> 250..272

<400> 1
Met Asp Phe Trp Leu Trp Pro Leu Tyr Phe Leu Pro Val Ser Gly Ala
1               5                   10                  15
Leu Arg Ile Leu Pro Glu Val Lys Val Glu Gly Glu Leu Gly Gly Ser
            20                  25                  30
Val Thr Ile Lys Cys Pro Leu Pro Glu Met His Val Arg Ile Tyr Leu
        35                  40                  45
Cys Arg Glu Met Ala Gly Ser Gly Thr Cys Gly Thr Val Val Ser Thr
    50                  55                  60
Thr Asn Phe Ile Lys Ala Glu Tyr Lys Gly Arg Val Thr Leu Lys Gln
65                  70                  75                  80
Tyr Pro Arg Lys Asn Leu Phe Leu Val Glu Val Thr Gln Leu Thr Glu
                85                  90                  95
Ser Asp Ser Gly Val Tyr Ala Cys Gly Ala Gly Met Asn Thr Asp Arg
            100                 105                 110
Gly Lys Thr Gln Lys Val Thr Leu Asn Val His Ser Glu Tyr Glu Pro
        115                 120                 125
Ser Trp Glu Glu Gln Pro Met Pro Glu Thr Pro Lys Trp Phe His Leu
    130                 135                 140
Pro Tyr Leu Phe Gln Met Pro Ala Tyr Ala Ser Ser Ser Lys Phe Val
145                 150                 155                 160
Thr Arg Val Thr Thr Pro Ala Gln Arg Gly Lys Val Pro Pro Val His
                165                 170                 175
His Ser Ser Pro Thr Thr Gln Ile Thr His Arg Pro Arg Val Ser Arg
            180                 185                 190
Ala Ser Ser Val Ala Gly Asp Lys Pro Arg Thr Phe Leu Pro Ser Thr
        195                 200                 205
Thr Ala Ser Lys Ile Ser Ala Leu Glu Gly Leu Leu Lys Pro Gln Thr
    210                 215                 220
Pro Ser Tyr Asn His His Thr Arg Leu His Arg Gln Arg Ala Leu Asp
225                 230                 235                 240
Tyr Gly Ser Gln Ser Gly Arg Glu Gly Gln Gly Phe His Ile Leu Ile
                245                 250                 255
Pro Thr Ile Leu Gly Leu Phe Leu Leu Ala Leu Leu Gly Leu Val Val
            260                 265                 270

Lys Arg Ala Val Glu Arg Arg Lys Ala Leu Ser Arg Arg Ala Arg Arg
        275                 280                 285
Leu Ala Val Arg Met Arg Ala Leu Glu Ser Ser Gln Arg Pro Arg Gly
    290                 295                 300
Ser Pro Arg Pro Arg Ser Gln Asn Asn Ile Tyr Ser Ala Cys Pro Arg
305                 310                 315                 320
Arg Ala Arg Gly Ala Asp Ala Ala Gly Thr Gly Glu Ala Pro Val Pro
                325                 330                 335
Gly Pro Gly Ala Pro Leu Pro Pro Ala Pro Leu Gln Val Ser Glu Ser
            340                 345                 350
Pro Trp Leu His Ala Pro Ser Leu Lys Thr Ser Cys Glu Tyr Val Ser
        355                 360                 365
Leu Tyr His Gln Pro Ala Ala Met Met Glu Asp Ser Asp Ser Asp Asp
    370                 375                 380
Tyr Ile Asn Val Pro Ala
385                 390

<210> 2
<211> 107
<212> PRT
<213> Homo sapiens

<220>
<223> Ig-like domain of FcÎ¼R

<400> 2
Arg Ile Leu Pro Glu Val Lys Val Glu Gly Glu Leu Gly Gly Ser Val
1               5                   10                  15
Thr Ile Lys Cys Pro Leu Pro Glu Met His Val Arg Ile Tyr Leu Cys
            20                  25                  30
Arg Glu Met Ala Gly Ser Gly Thr Cys Gly Thr Val Val Ser Thr Thr
        35                  40                  45
Asn Phe Ile Lys Ala Glu Tyr Lys Gly Arg Val Thr Leu Lys Gln Tyr
    50                  55                  60
Pro Arg Lys Asn Leu Phe Leu Val Glu Val Thr Gln Leu Thr Glu Ser
65                  70                  75                  80
Asp Ser Gly Val Tyr Ala Cys Gly Ala Gly Met Asn Thr Asp Arg Gly
                85                  90                  95
Lys Thr Gln Lys Val Thr Leu Asn Val His Ser
            100                 105

<210> 3
<211> 139
<212> PRT
<213> Artificial Sequence

<220>
<223> fusion protein His10-Xa-3C-FcuR-IgI

<400> 3
Met Gly His His His His His His His His His His Ser Ser Gly His
1               5                   10                  15
Ile Glu Gly Arg His Met Gly Leu Glu Val Leu Phe Gln Gly Pro Gly
            20                  25                  30
Arg Ile Leu Pro Glu Val Lys Val Glu Gly Glu Leu Gly Gly Ser Val
        35                  40                  45
Thr Ile Lys Cys Pro Leu Pro Glu Met His Val Arg Ile Tyr Leu Cys
    50                  55                  60
Arg Glu Met Ala Gly Ser Gly Thr Cys Gly Thr Val Val Ser Thr Thr
65                  70                  75                  80
Asn Phe Ile Lys Ala Glu Tyr Lys Gly Arg Val Thr Leu Lys Gln Tyr
                85                  90                  95
Pro Arg Lys Asn Leu Phe Leu Val Glu Val Thr Gln Leu Thr Glu Ser

            100                 105                 110
Asp Ser Gly Val Tyr Ala Cys Gly Ala Gly Met Asn Thr Asp Arg Gly
        115                 120                 125
Lys Thr Gln Lys Val Thr Leu Asn Val His Ser
    130                 135

<210> 4
<211> 264
<212> PRT
<213> Artificial Sequence

<220>
<223> fusion protein His10-Xa-3C-FcuR-ECD

<400> 4
Met Gly His His His His His His His His His His Ser Ser Gly His
1               5                   10                  15
Ile Glu Gly Arg His Met Gly Leu Glu Val Leu Phe Gln Gly Pro Gly
            20                  25                  30
Arg Ile Leu Pro Glu Val Lys Val Glu Gly Glu Leu Gly Gly Ser Val
        35                  40                  45
Thr Ile Lys Cys Pro Leu Pro Glu Met His Val Arg Ile Tyr Leu Cys
    50                  55                  60
Arg Glu Met Ala Gly Ser Gly Thr Cys Gly Thr Val Val Ser Thr Thr
65                  70                  75                  80
Asn Phe Ile Lys Ala Glu Tyr Lys Gly Arg Val Thr Leu Lys Gln Tyr
                85                  90                  95
Pro Arg Lys Asn Leu Phe Leu Val Glu Val Thr Gln Leu Thr Glu Ser
            100                 105                 110
Asp Ser Gly Val Tyr Ala Cys Gly Ala Gly Met Asn Thr Asp Arg Gly
        115                 120                 125
Lys Thr Gln Lys Val Thr Leu Asn Val His Ser Glu Tyr Glu Pro Ser
    130                 135                 140
Trp Glu Glu Gln Pro Met Pro Glu Thr Pro Lys Trp Phe His Leu Pro
145                 150                 155                 160
Tyr Leu Phe Gln Met Pro Ala Tyr Ala Ser Ser Ser Lys Phe Val Thr
                165                 170                 175
Arg Val Thr Thr Pro Ala Gln Arg Gly Lys Val Pro Pro Val His His
            180                 185                 190
Ser Ser Pro Thr Thr Gln Ile Thr His Arg Pro Arg Val Ser Arg Ala
        195                 200                 205
Ser Ser Val Ala Gly Asp Lys Pro Arg Thr Phe Leu Pro Ser Thr Thr
    210                 215                 220
Ala Ser Lys Ile Ser Ala Leu Glu Gly Leu Leu Lys Pro Gln Thr Pro
225                 230                 235                 240
Ser Tyr Asn His His Thr Arg Leu His Arg Gln Arg Ala Leu Asp Tyr
                245                 250                 255
Gly Ser Gln Ser Gly Arg Glu Gly
            260

<210> 5
<211> 125
<212> PRT
<213> Homo sapiens

<220>
<223> C-terminal domain of Faim 3/Toso

<400> 5
Glu Tyr Glu Pro Ser Trp Glu Glu Gln Pro Met Pro Glu Thr Pro Lys
1               5                   10                  15
Trp Phe His Leu Pro Tyr Leu Phe Gln Met Pro Ala Tyr Ala Ser Ser
            20                  25                  30

Ser Lys Phe Val Thr Arg Val Thr Thr Pro Ala Gln Arg Gly Lys Val
        35                  40                  45
Pro Pro Val His His Ser Ser Pro Thr Thr Gln Ile Thr His Arg Pro
    50                  55                  60
Arg Val Ser Arg Ala Ser Ser Val Ala Gly Asp Lys Pro Arg Thr Phe
65                  70                  75                  80
Leu Pro Ser Thr Thr Ala Ser Lys Ile Ser Ala Leu Glu Gly Leu Leu
                85                  90                  95
Lys Pro Gln Thr Pro Ser Tyr Asn His His Thr Arg Leu His Arg Gln
            100                 105                 110
Arg Ala Leu Asp Tyr Gly Ser Gln Ser Gly Arg Glu Gly
        115                 120                 125

<210> 6
<211> 43
<212> DNA
<213> Artificial Sequence

<220>
<223> Primer

<400> 6
ctctttcagg gacccgggag gatcctccca gaagtaaagg tag                         43


<210> 7
<211> 42
<212> DNA
<213> Artificial Sequence

<220>
<223> Primer

<400> 7
agttagctag ggcccgggtc aactgtggac attcagggtg ac                          42


<210> 8
<211> 43
<212> DNA
<213> Artificial Sequence

<220>
<223> Primer

<400> 8
agttagctag ggcccgggtc agccttccct cccagactgt gag                         43

## Description

The present invention relates to the field of diagnostic or analytic methods. In particular, the inventors teach that an Fc receptor for IgM heavy chains (FcµR), in particular, Faim 3/Toso and also CD351 (Fcµ/αR), or a functional fragment thereof, which preferably comprises the Ig-like domain of Faim 3/Toso, may be used for *in vitro* quantification of IgM or IgA antibodies, preferably, IgM, in the form of immune complexes, i.e., complexes formed by antigen and specific antibody. These immune complexes may be formed by mixing an antigen with a sample comprising antibodies. A method for detection and quantification of IgA or IgM antibodies in the form of immune complexes is also provided. This can be useful for diagnosis of infections, e.g., with a virus.

IgM antibodies belong to the first line of defense of the specific immune system against pathogens. IgM antibodies are produced by B-cells before class switch to IgG. Detection of IgM is thus especially suited for early diagnosis of an infection. IgM have a different structure from IgG, which leads to a particularly high avidity of the antibody, as each IgM has 10 antigen binding sites instead of 2 for IgG.

IgM is classically detected by the IgM antibody-capture (MAC) approach, i.e., a test serum of a patient is applied to a microtitration plate previously coated with anti-human IgM, followed by application of a labelled antigen (Duermeyer W, Wielaard F. et al., 1979; Ong et al., 1989, Vorndam V., Kuno G., 1997, Balmaseda A., Guzman, M.G.et al., 2003, Vasquez, Hafner et al., 2007).

In light of this, the inventors address the problem of providing improved means for detection of IgM and/or IgA antibodies.

The problem faced by the inventors is solved by the subject matter of the claims.

In one aspect, the invention provides use of an FcµR, which, preferably, is immobilized on a solid support, for *in vitro* quantifying antibodies in the form of immune complexes.

More than thirty years ago, IgM receptors (Moretta et al., 1977, Hoover et al., 1981) and an IgA receptor (Ohno et al., 1990) had already been described on human B or T cells, but sequence data were not available at that time.

Surprisingly, the correct DNA sequence of an FcµR had already been published in 1998, and the protein found on Jurkat T cells had been named Fas apoptotic inhibitory molecule 3 (FAIM3) or TOSO (Hitoshi et al., 1998). However, the former designation was incorrect, as the molecule did not induce apoptosis. The misinterpetation was due to use of mouse IgM anti-Fas mAb (CH11), which induced Fas-mediated apoptosis. The identity of an FcµR with the well known FAIM3 molecule, which was also named Toso, was reported in 2009, when a cDNA (1,173-bp open reading frame) responsible for IgM binding (Kubagawa et al., 2009) was sequenced and compared to other known sequences in the database. The FcµR is the only Fc receptor (FcR) constitutively expressed on T cells.

The FcµR consists of a 107-aa V-set Ig-like domain responsible for IgM binding, an additional 127-aa extra cellular region with no known domain structure, a 21-aa transmembrane segment containing a charged His- residue, and a relatively long (118-aa) cytoplasmic tail (Kubagawa et al., 2014). FcµR ligation with preformed IgM immune complexes induced phosphorylation of both Tyr and Ser residues of the 60 kD receptor. Curiously, mouse IgM bound better to the human FcµR than human IgM.

A soluble serum FcµR resolved as a ∼40 kDa protein, distinct from the cell surface FcµR of ∼60 kDa, is produced by both B and non-L B cells by a splicing process of the FcµR cDNA.

Among the multitude of FcRs, there are also IgA-binding and IgA/ IgM-binding receptors, which have been found on B-lymphocytes, and which are of special interest in the present context.

The Fc receptor designated Fcα/µR or CD351 has been characterized in mice and humans, and binds both IgM and IgA with intermediate or high affinity. It can therefore be considered a further FcµR. Human Fcα/µR is constitutively expressed on the majority of B lymphocytes and macrophages (Shibuya et al., 2000). To further characterize this receptor, it was expressed on the surface of the 293T cell line and affinity of the interactions of the receptor with IgA and IgM was measured (Yoo et al., 2011, van der Boog et al., 2002).

In addition, a human myeloid-specific IgA Fc receptor has been described (FcαRI, CD89). It is also encoded in the leukocyte receptor complex of rats (Maruoka et al., 2004). FcαRI or CD89 is expressed on the surface of eosinophils, neutrophils, dendritic cells, monocytes and macrophages.

In the context of the invention, the FcµR may be Faim 3/Toso or a functional fragment thereof capable of binding to immune complexes, or CD351 or a functional fragment thereof capable of binding to immune complexes are of special interest. If the FcµR is Faim 3/Toso or a fragment thereof, the antibody is of the IgM class. If FcµR is CD351 or a fragment thereof, the antibody is of the IgM or IgA class.

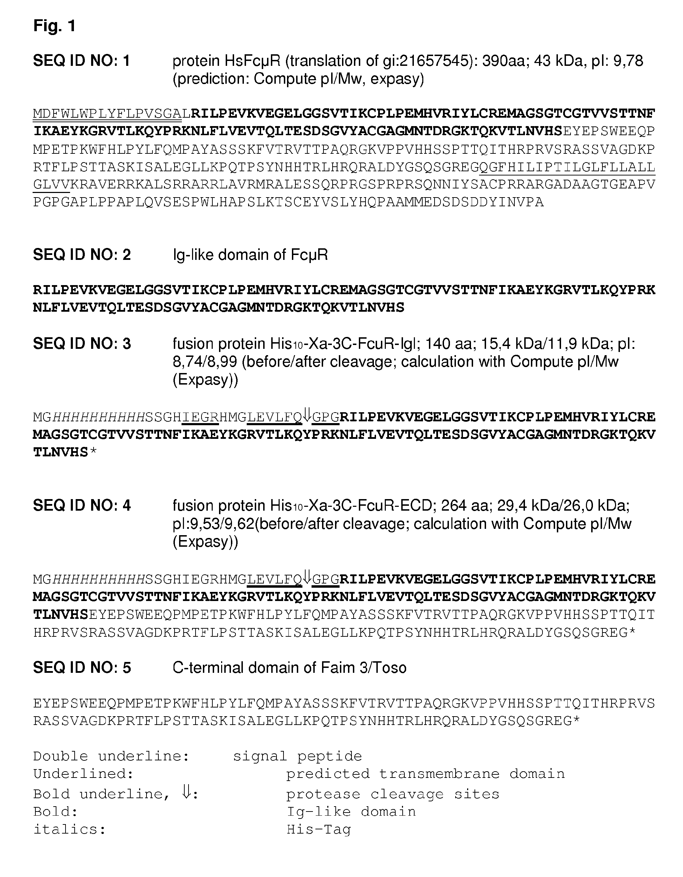

Preferably, the FcµR is Faim 3/Toso or a functional fragment thereof capable of binding to immune complexes. It may comprise an amino acid sequence having at least 80%, at least 90%, at least 95%, at least 99% or 100% sequence identity with, preferably, human Faim 3/Toso (SEQ ID NO: 1). (See Fig. 1). The fragment of the FcµR preferably comprises an amino acid sequence having at least 80%, at least 90%, at least 95%, at least 99% or 100% sequence identity with the functional Faim 3/Toso fragment, in particular, human Faim 3/Toso fragment. The area of the defined sequence identity is capable of binding to immune complexes, e.g., as tested in the examples below.

The functional fragment comprises the Ig-like domain of Faim 3/Toso, which preferably has SEQ ID NO: 2 or at least 80%, more preferable, at least 85%, at least 90%, at least 95% or at least 99% amino acid identity to SEQ ID NO: 2, or which varies from SEQ ID NO: 2 in only one or two amino acids. It may also consist or essentially consist of the Ig-like domain. In the context of this application, "essentially consist" means that at least 70%, preferably, at least 75%, at least 77%, at least 80%, at least 90% at least 95%, or at least 99% of the proteins amino acids of the protein correspond to the Ig-like domain of Faim 3/Toso. Amino acids not corresponding to the Ig-like domain of Faim 3 /Toso may be amino acids of a tag for purification, e.g., a His-Tag, or a linker, e.g., incorporating a cleavage site.

Most preferably, the fragment does not comprise the complete extracellular domain of Faim 3/Toso. In particular, it does not comprise SEQ ID NO: 5, i.e., the part of Faim 3/Toso C-terminal to the Ig-like domain. Preferably, the functional fragment of Faim 3/Toso does not comprise more than 10%, more than 20%, more than 30%, more than 40%, more than 50%, more than 60%, more than 70%, more than 80%, more than 90% of the part of Faim 3/Toso C-terminal to the Ig-like domain.

The invention also relates to a protein comprising SEQ ID NO: 2 or having at least 80%, at least 85%, at least 90%, at least 95% or at least 99% amino acid identity thereto, wherein the protein preferably does not comprise a sequence having more than 10%, more than 20%, more than 30%, more than 40%, more than 50%, more than 60%, more than 70%, more than 80%, or more than 90% amino acid identity to SEQ ID NO: 5, or wherein the protein does not comprise SEQ ID NO: 5. A protein essentially consisting of SEQ ID NO: 2 is provided, which may be used in the methods of the present invention.

Preferably, the protein fragment does not comprise the intracellular or transmembrane region of Faim 3/Toso.

In one embodiment, the FcµR does not comprise a His-Tag, in particular, if the sample may comprise anti-malaria antibodies. Anti-malaria-antibodies have been shown to be able to cross-react with His-tags under certain conditions. A His-Tag may be used for purification of the FcµR, but it is preferably linked to the FcµR through a protease cleavage site, so the His-Tag may be cleaved off, if needed, for example, for detection of anti-malaria antibodies. If the His-Tag does not interfere with detection of immune complexes, it may be incorporated in the final product.

His₁₀-Xa-3C-FcµR-Igl (SEQ ID NO: 3) or a protein having at least 80%, at least 90%, at least 95%, or at least 99% amino acid identity to SEQ ID NO: 3 is a preferred FcµR of the present invention. The inventors surprisingly showed significant advantages compared to a fragment comprising the complete extracellular domain of Faim 3/Toso (SEQ ID NO: 4).

Alternatively, the FcµR may be CD351 or a functional fragment thereof capable of binding to immune complexes. It may comprise an amino acid sequence having at least 80%, at least 90%, at least 95%, at least 99% or 100% sequence identity with, preferably, human CD351. The fragment of the FcµR preferably comprises an amino acid sequence having at least 80%, at least 90%, at least 95%, at least 99% or 100% sequence identity with the functional CD351 fragment, in particular, human CD351 fragment. The area of the defined sequence identity is capable of binding to immune complexes, e.g., as tested in the examples below.

Recombinant FcµRs are available. For example, they can be expressed in E. coli. Expression in mammalian expression systems, e.g., COS cells or CHO cells, is also possible. Accordingly, FcµR may be glycosylated or non-glycosylated. The inventors have shown that non-glycosylated forms of Faim/Toso such as SEQ ID NO: 2, in particular, SEQ ID NO: 3, were able to bind the immune complexes and performed well in the method of the invention.

The IC ELISA experiments with various FcµR molecules performed by the inventors show that Faim 3/Toso, in particular a fragment of Faim 3/Toso based on the Ig-like domain and not including the complete part of Faim 3/Toso C-terminal to the Ig-like domain is particularly well suited to bind the immune complexes for their detection.

The immune complexes comprise an antigen and an antibody specific for said antigen, thus allowing quantification of specific antibodies. The immune complexes are preferably formed by mixing an antigen with a sample comprising antibodies.

The antigen is detectable, i.e., preferably, it is a labelled antigen, e.g., an enzyme labelled antigen (ELA), which allows for easy detection of the immune complexes. The enzyme may be peroxidase, e.g., horseradish peroxidase. Alkaline phosphatase may also be used. Other labelling methods, like biotinylation or labelling with a fluorescent moiety such as FITC, PE or a fluorescent protein, may also be used. Alternatively, the antigen may be unlabeled if it comprises more than one epitope. A second, a labelled antibody, preferably monoclonal, may then be used to detect and quantify the immune complexes. If the label is biotin, detection may be performed by adding an avidin- or streptavidin-labelled enzyme in a further step. Addition of a substrate of the enzyme, as well-known in the state of the art, allows for quantification of label, and consequently, of antibody in immune complexed form.

The immune complexes may thus be quantified to determine or to quantify presence of specific antibodies in the sample.

The antibodies which form the immune complexes will be of the IgM or IgA class, preferably, IgM.

The composition comprising antibodies that is analysed is a sample, e.g., a biological sample, such as a sample from a patient. It may also be a sample from a subject known to have, or not to have the antibodies of interest, e.g., as a positive or negative control. Preferably, samples from a subject and a positive and/or negative control are analysed in the same test to allow direct comparison. The sample may be derived from blood (e.g., plasma or, preferably, serum), stool, saliva, sputum, or liquor cerebrospinalis. The sample, e.g., serum, may be diluted before contacting the FcµR, e.g., diluted 1:10 or more, or 1:100 or more, e.g., in a suitable buffer such as PBS or citrate buffer, or it may be otherwise processed, e.g., by removing certain components, such as cells from blood or by concentration.

The sample may be from a human subject or an animal, e.g., a goat, sheep, horse, cattle, a bird, e.g., chicken, or a rodent such as mouse, rat, rabbit, guinea pig or hamster. The immune complexes may thus be formed by an antigen such as an enzyme-linked antigen and by antibodies which are derived from a species selected from the group comprising human, goat, sheep, horse, cattle, an avian species, e.g., chicken, or a rodent such as mouse, rat, rabbit, guinea pig or hamster. In this context, the FcµR preferably is a human FcµR.

The inventors were able to show that human FcµR, in particular, a fragment comprising the Ig-like domain, has a surprisingly wide interspecies reactivity. For example, murine IgM antibodies can also be detected.

In one embodiment, the immune complexes are formed by addition of antigen to the sample comprising antibodies before contacting the sample with FcµR, i.e., the FcµR binds to preformed immune complexes.

However, the addition of antigen to the sample comprising antibodies is preferably performed in the presence of the FcµR, i.e., the immune complexes are formed in the presence of the FcµR, e.g., in the presence of Faim 3/Toso or a functional fragment thereof. The inventors found that this improves sensitivity of the test in comparison with a test wherein first, the antibodies are bound by the FcµR, and, after a washing step, the antigen is added to form immune complexes. It may also save time in comparison to preformation of immune complexes. The contacting of the FcµR with the antibody may thus be essentially simultaneous with contacting of the antibodies, which are to be quantified, with the labelled antigen.

The invention also provides a method for detecting immune complexes, in particular, a method for in vitro quantifying antibodies in the form of immune complexes, comprising
a) coating a solid support with an FcµR under conditions suitable for binding of the FcµR to the solid support,
b) incubating the solid support with a composition comprising an antibody and an antigen, wherein said antibody and said antigen are capable of forming an immune complex, and,
c) washing the solid support, and then
d) determining quantity of the bound antigen within the immune complex, and thereby determining quantity of the bound immune complex.

The solid support may be, e.g., a plastic plate, in particular a plastic plate suitable for ELISA, as well known in the state of the art, e.g., a polystyrene plate having wells, normally, 96 wells. The solid support may also be, e.g., a coverslip, a column or a resin suitable for filling a column, or a bead, e.g., a glass bead, plastic bead, or a magnetic bead. Preferably, the solid support is washed after step a. A blocking step, e.g., with a buffer such as PBS comprising bovine serum albumin (BSA) or another irrelevant protein can also be contemplated.

In one embodiment, the composition of step b comprises an immune complex formed by the antigen and the antibody. The immune complex formed by an antigen and an Ig antibody may be formed before incubation with the FcµR on the solid support, e.g., by adding an antigen to a sample comprising, preferably, IgM antibody specific to the antigen. The inventors have shown that a washing step of the solid between incubation of the FcµR with the antibody and incubation with the antigen strongly decreases sensitivity. In comparison, it is better to incubate the FcµR with antigen and antibody simultaneously, i.e., wherein the antigen and antibody have already formed immune complexes, or wherein the immune complexes form in the presence of FcµR. Formation of the immune complex in the presence of FcµR is possible and leads to at least equivalent results. This may save time in comparison with previous formation of the immune complexes. Sufficient incubation times for formation of the complexes and equilibration of binding should be used, as appropriate for the conditions chosen.

Suitable conditions for formation of the immune complexes and binding of the FcµR to the support are described, e.g, in the examples, but are also evident to one of skill in the art. The incubation steps are preferably for one to 48 hours, each. Preferably, the incubation, in particular incubation of the coated support with the antibody and/or immune complexes is carried out for at least 8 hours, at least 12 hours, at least 24 hours, or at least 48 hours, or overnight. In comparison to indirect ELISAs or IIF, relatively long incubation times are thus required for optimal results. One explanation for the finding that the results are better with longer incubation times, might be that the multivalent high molecular immune complexes need time to compete with and partially displace mlgM or aglgM at the FcµR molecules.

Incubation may be on ice, at 4-8°C or at room temperature, e.g., at 20°C-25°C. Optimal incubation times and temperatures influence each other, and can be determined according to the needs of the assay and the characteristics of the reagents. The inventors were able to show that at higher incubation temperatures (e.g., 30-40°C, preferably, about 37°C), incubation time can be shortened to several hours (e.g., at least 2 hours, at least 4 hours, at least 8 hours or at least 12 hours. Thus, the test also fulfills the time requirements for an acute diagnostics test.

Incubation may be in a buffer, e.g., PBS, optionally comprising a non-ionic detergent like Tween and/or a blocking agent such as BSA. Preferably, conditions are chosen, which correspond to conditions in assays known in the state of the art for detecting immune complexes with RF.

Washing steps may, e.g., be carried out with PBS/Tween, optionally comprising BSA or another protein different from the antigen or the antibody. The quantity, presence or absence of the immune complex may be determined as known in the state of the art, e.g., as described for an enzyme-linked antigen in the examples below. Alternatively, a labelled antibody directed to the antigen may be employed for detection. The quantity of label detected correlates with the quantity of antibody in the form of immune complexes, i.e., antibody specific for the antigen.

Further details have been described herein with regard to the use of the FcµR, which details also form preferred embodiments in the method of the invention.

The invention also provides a method for diagnosing an infection with a pathogen in a subject, e.g., a virus infection, wherein the method for detecting immune complexes described above is carried out, wherein the composition comprising an antibody is a sample from the subject, and wherein the antigen which can be specifically bound by said antibody is an antigen derivable from the pathogen. The pathogen may, e.g., be a virus, such as Crimean-Congo hemorrhagic fever (CCHF) virus, West Nile (WN) virus, dengue virus, Epstein-Barr virus or Tick-borne encephalitis (TBE) virus. The antigen preferably is major antigen of the respective pathogen, for example a surface (glyco-) protein, or a poly- or oligosaccharide structure thereof, such as an envelope protein of a virus, like the flavivirus envelope protein E. Alternatively, the antigen may be a capsid antigen. For CCHF virus, CCHFV nucleoprotein NP is preferably used. The antigen may be recombinantly prepared. As described before, the antigen may be labelled, e.g. with biotin or an enzyme such as horseradish peroxidase, to facilitate detection.

Using a sample having a known concentration of antibody as a standard, the amount of antibody in the sample can easily be determined by the skilled person. The amount of antibody can then be compared, e.g., with known amounts of healthy subjects, of subjects having an acute infection with the pathogen. Presence of an antibody specific to an antigen derived from a pathogen typically indicates that the subject is infected with the pathogen. A high amount of antibody may e.g. indicate an acute stage of the infection. The concentration of antibodies in one subject may also be followed over time, which may allow conclusions regarding the course of the infection, or the level of immunity after a vaccination.

The invention provides several advantages over the state of the art. For example, it can be assumed that detection of specific IgM can be performed at lower concentrations, i.e., earlier in the course of an infection, as the specific IgM are bound and detected after formation of an immune complex with their specific antigen. In comparison, in state of the art µ capture assays, all IgM in a sample are bound to the support, but only specific IgM can generate a signal.

The invention is illustrated, but not limited by the specific examples below. All cited literature is herewith incorporated by reference.

### Figure legends

**Fig. 1** Sequences of different variants of FcµR are shown:
- SEQ ID NO: 1: protein Hs FcµR (translation of gi:21657545): 390aa; 43 kDa, pI: 9,78 (prediction: Compute pI/Mw, Expasy)
- SEQ ID NO: 2: Ig-like domain of FcµR
- SEQ ID NO: 3: fusion protein His₁₀-Xa-3C-FcµR-lgl; 140 aa; 15,4 kDa/11,9 kDa; pI: 8,74/8,99 (before/after cleavage; calculation with Compute pI/Mw, Expasy)
- SEQ ID NO: 4: fusion protein His₁₀-Xa-3C- FcµR-ECD; 264 aa; 29,4 kDa/26,0 kDa; pI:9,53/9,62 (before/after cleavage; calculation with Compute pI/Mw , Expasy)
- SEQ ID NO: 5: C-terminal domain of Faim 3/Toso
- Double underline:: signal peptide
- Underlined:: predicted transmembrane domain
- Bold underline:: protease cleavage site
- Bold:: Ig-like domain
- Italics:: His-Tag

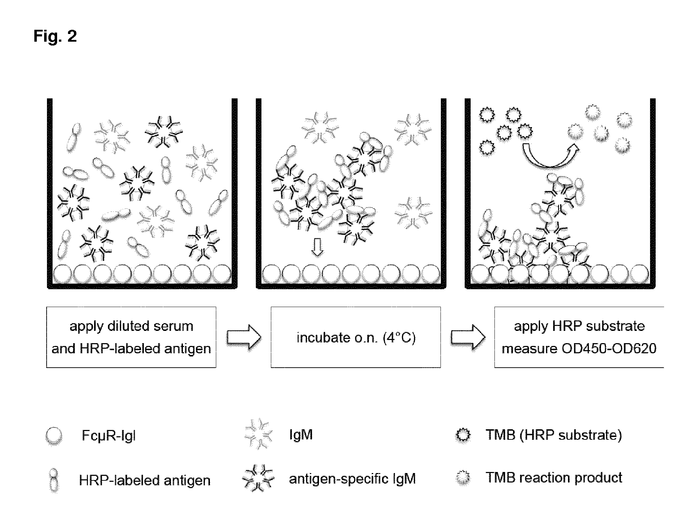

**Fig. 2** Schematic of the IgM-ICB ELISA procedure. Plates are coated with bacterially expressed FcµR, e.g., HsFcµR fragment such as His₁₀-3C-HsFcµR-Igl. Serum and labelled recombinant antigen (e.g., labelling with HRP) are applied to the wells. During the following incubation time, immune complexes form and bind to the FcµR. After plate washing, bound immune complexes are detected, e.g. by using the colorimetric HRP-substrate TMB. In contrast to the conventional µ-capture IgM ELISA technique, only IgM molecules specifically recognizing the recombinant antigen are bound to the plate, improving test sensitivity.

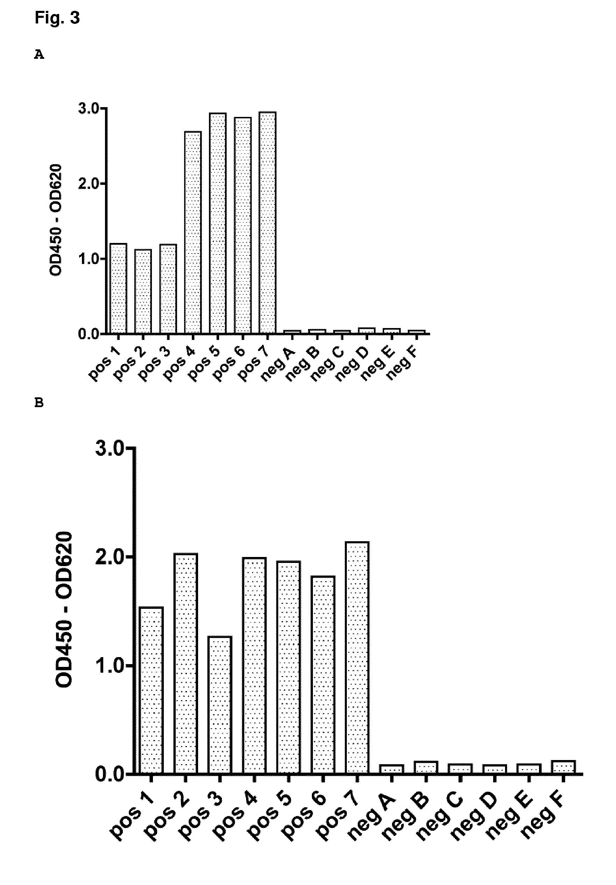

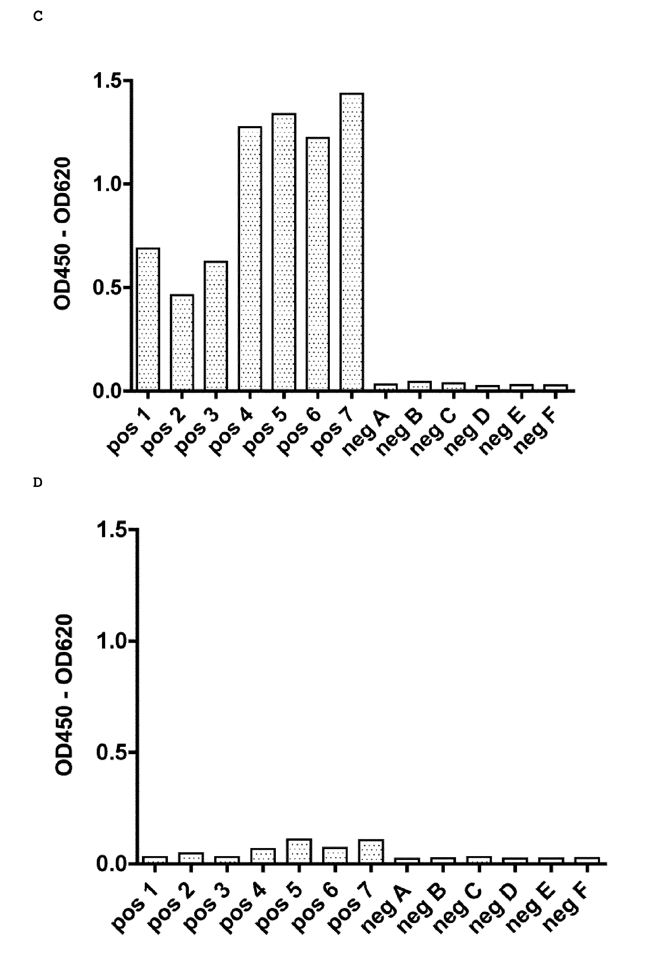

**Fig. 3** Comparison of IgM-ICB-ELISA procedure with state of the art µ-capture ELISA.
(A) Analysis of anti-CCHFV-IgM positive sera using a commercially available µ-capture ELISA (VectoCrimea-CHF-IgM). Seven anti-CCHFV-IgM positive sera (confirmed by immunofluorescence analysis, titers sera pos 1, pos 2, pos 3: < 640; titers sera pos 4, pos 5, pos 6, pos 7: > 640) and six negative sera were analyzed using the VectoCrimea-CHF-Kit (VectorBest) according to the manufacturer's instructions (dilution of sera: 1:100; TMB reaction: RT, 25 min).
(B) Analysis of anti-CCHFV-IgM positive sera with the IgM-ICB-ELISA. Sera (final dilution 1:100) and recombinantly produced, HRP-labelled antigen (final dilution 1:32.000) were co-incubated on His10-3C-HsFcµR-coated plates for 24 h at 4°C. After washing, TMB substrate was added and plates were incubated for 10 min at RT before stopping the reaction.
(C) Analysis of anti-CCHFV-IgM positive sera using a µ-capture ELISA. Plates coated with anti-human IgM were incubated with sera (diluted 1:100) for 1 h at RT. After washing, plates were incubated with recombinantly produced HRP-labelled antigen (CCHFV-NP, dilution 1:50.000) for 1 h at RT. After washing, TMB substrate was added and plates were incubated for 10 min at RT before stopping the reaction.
(D) His10-3C-HsFcµR-Igl binds only weakly to non-complexed IgM. Plates coated with His10-3C-HsFcµR-Igl were incubated with sera (diluted 1:100) for 1 h at RT. After washing, plates were incubated with recombinantly produced, HRP-labelled antigen (CCHFV-NP, dilution 1:50.000) for 1 h at RT. After washing, TMB substrate was added and plates were incubated for 10 min at RT before stopping the reaction.

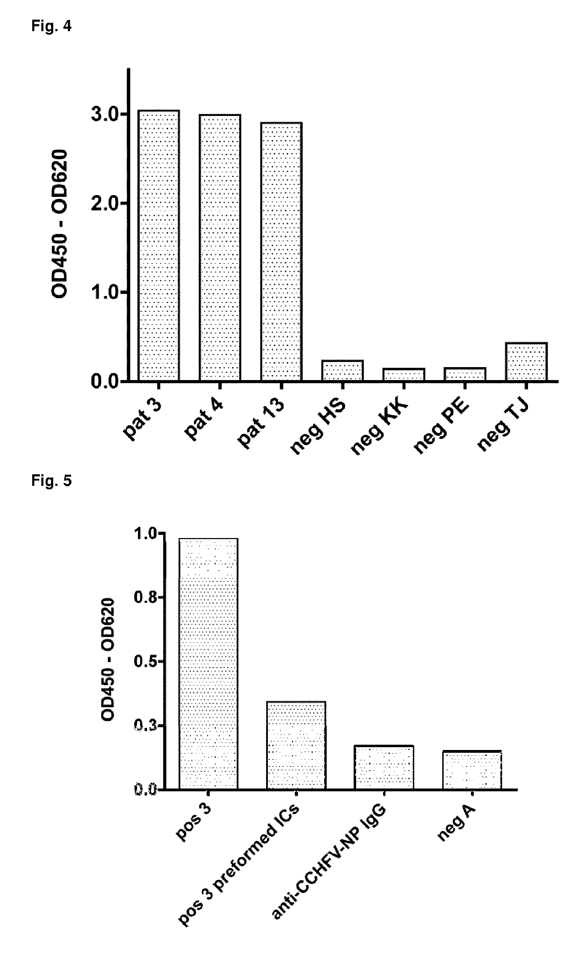

**Fig. 4** Analysis of anti-Lassa-IgM positive sera with an IgM-ICB-ELISA. Sera (final dilution 1:100 or 1:10) and biotinylated Lassa nucleoprotein (final dilution 1:1000) were co-incubated on His10-3C-HsFcµR-coated plates for 24 h at 4°C. HRP-labeled streptavidine at a dilution of 1:10.000 was added for one hour. Upon renewed washing the test was stained using TMB.

**Fig. 5****.** Immobilized His10-3C-HsFcµR-Igl binds to IgM/antigen immune complexes but not to IgG/antigen immune complexes. Preformed IgM/antigen complexes are bound less efficiently than nascent IgM/antigen immune complexes. Sera (final dilution 1:100) pos 3 and neg A or mouse monoclonal anti-CCHFV-NP IgG (final dilution 1:20.000) were co-incubated on His10-3C-HsFcµR-coated plates for 24 h at 4°C with recombinantly produced, HRP-labelled antigen (CCHFV-NP, final dilution 1:32.000). Alternatively, serum pos 3 was co-incubated with recombinantly produced, HRP-labelled CCHFV-NP in a test tube for 19 h at 4°C. Preformed immune complexes were then added to a well of the His10-3C-HsFcµR-Igl-coated plate and incubated for 5 h at 4°C. After washing, TMB substrate was added and plates were incubated for 10 min at RT before stopping the reaction.

### Examples

### Material and Methods

### Generation of prokaryotic expression vectors

Fragments encoding either the immunoglobulin-like domain (Igl, primers FcµR-IF-F (5' - CTCTTTCAGGGACCCGGGAGGATCCTCCCAGAAGTAAAGGTAG - 3', 360 bp, SEQ ID NO:6) and FcµR-IF-R1 (5' - AGTTAGCTAGGGCCCGGGTCAACTGTGGACATTCAGGGTGAC - 3,', SEQ ID NO:7)) or the complete extracellular domain (ECD, primers FcµR-IF-F and FcµR-IF-R2 (5' - AGTTAGCTAGGGCCCGGGTCAGCCTTCCCTCCCAGACTGTGAG - 3'), 735 bp, SEQ ID NO:8) of HsFcµR were amplified using the plasmid pGEM-T-HsFcµR (Human FAIM3 Gene cDNA Clone, Sino Biological) as a template. Fragments were cloned into the prokaryotic expression vector pJC45-CD32-3C-His (Emmerich et al., 2013) cut with SmaI by In-Fusion-Cloning (Clontech). The resulting plasmids pJC45-His₁₀-3C-HsFcµR-Igl and pJC45-His₁₀-3C-HsFcµR-ECD encode fusion proteins of 15 kDa and 29 kDa, respectively, comprising an N-terminal 10 x His-Tag followed by a 3C protease cleavage site and the respective HsFcµR fragment.

A cDNA fragment encoding the nucleoprotein (NP) of CCHFV strain Afg09-2990 (ADQ57288, Ölschläger et al., 2011) was cloned into the prokaryotic expression vector pOPINJ (Berrow et al., 2007). The resulting plasmid pOPINJ-CCHFV-NP encodes a 82 kDa fusion protein comprising an N-terminal 6 x His-Tag followed by GST (26 kDa), a 3C protease cleavage site and the CCHFV-NP (54 kDa).

### Recombinant expression and purification of HsFcµR fragments ECD and Igl

Plasmids pJC45-His₁₀-3C-HsFcµR-Igl and pJC45-His₁₀-3C-HsFcµR-ECD were transformed into *E.coli* pAPlacI^{Q} cells. Cells were grown at 37°C to an optical density at 600 nm of 0.5 - 0.6 in LB medium supplemented with 50 µg/ml kanamycin and 100 µg/ml ampicillin, then expression of recombinant proteins was induced by the addition of IPTG (final concentration 1 mM). Bacteria were incubated further over night at 18°C and then harvested by centrifugation (4000 x g, 10 min, 4°C). Cells were resuspended in PBS pH 7.4 supplemented with 1 mM PMSF, 10 mM EDTA, 0.25 mg/ml lysozyme and 0.01 mg/ml DNAse and lyzed for 30 min at RT. After sonification, the lysate was centrifuged for 20 min at 12000g and 4°C. Inclusion bodies were washed twice by homogenization in PBS pH 7.4 supplemented with 0.5 % Triton X-100 followed by centrifugation (12000 x g, 20 min, 4°C). Washed inclusion bodies were solubilized in 50 mM Tris-HCl ph 7.8 / 6 M guanidine hydrochloride for 50 min at 4°C. After mixing 1:1 with Binding Buffer (10 mM Tris-HCl pH 8.0, 6M urea, 50 mM NaH₂PO₄, 5 mM imidazole, 150 mM NaCl, 1 % glycerol) the solubilisate was loaded onto Ni-NTA agarose (Qiagen). After washing the column twice with Wash Buffer (50 mM KH₂PO₄, 300 mM KCI, 6 M urea, 10 mM imidazole, pH 8.0), protein was eluted with Elution Buffer (50 mM KH₂PO₄, 300 mM KCI, 6 M urea, 250 mM imidazole, pH 8.0). Subsequently, refolding was performed by rapid dilution. Therefore, DTT was added to the eluate at a final concentration of 10 mM and the sample was incubated for 30 min on ice. Then, the sample was diluted drop-wise into the 40fold volume of Refolding Buffer (100 mM Tris pH 8.5, 0.3 M arginine, 150 mM NaCl, 5 mM GSH, 0.5 mM GSSG, 0.1 M PMSF, 0.01% NaN₃) and gently stirred at 4°C for three days. Afterwards, the refolded protein was concentrated using centrifugal filter units (membrane NMWL 3 kDa, Amicon).

### Recombinant expression and purification of CCHFV antigen

The plasmid pOPINJ-CCHFV-NP was transformed into *E.coli* pAPlacI^{Q} cells. Cells were grown at 37°C to an optical density at 600 nm of 0.5 - 0.6 in LB medium supplemented with 50 µg/ml kanamycin and 100 µg/ml ampicillin, then expression of the CCHFV-NP fusion protein was induced by the addition of IPTG (final concentration 1 mM). Bacteria were incubated further over night at 18°C and then harvested by centrifugation (4000 x g, 20 min, 4°C). Cells were resuspended in lysis buffer (50 mM NaH₂PO₄, 300 mM NaCl, 10 mM imidazole; pH 8.0) supplemented with 1 mM PMSF and 1 mg/ml lysozyme and incubated on ice for 30 min before sonification. After sonification, DNAse was added to a final concentration of 10 µg/ml. The lysate was incubated for 15 - 30 min on ice and then centrifuged at 10000 x g for 20 min at 4°C. The resulting supernatant was incubated with pre-equilibrated Ni-NTA-agarose for 1h at 4°C. The matrix was transferred to a column and the flow-through was discarded. After washing the column with a 5 fold volume of Wash Buffer 1 (50 mM NaH₂PO₄, 300 mM NaCl, 20 mM imidazole; pH 8.0) and a 5 fold volume of Wash Buffer 2 (50 mM NaH₂PO₄, 500 mM NaCl, 20 mM imidazole; pH 8.0), bound protein was eluted with Elution Buffer (50 mM NaH₂PO₄, 300 mM NaCl, 250 mM imidazole; pH 8.0).

The main fractions were pooled and the buffer was exchanged to Binding Buffer (50 mM NaH₂PO₄, 150 mM NaCl, 5 mM DTT; pH 7.2) using Zeba Spin Desalting Columns. Further purification was performed by binding to glutathione agarose, followed by on-column cleavage with 3C protease, and final purification by size exclusion chromatography.

### HRP-labeling of CCHFV antigen

### IgM µ-capture ELISA

VectoCrimea-CHF-IgM µ-capture ELISA (VectorBest, Russia) was carried out according to the manufacturer's instructions.

A CCHFV µ-capture test using CCHFV-NP^{HRP} was carried out using the same sequence of immunological reactions. In brief, plates coated with anti-human IgM were incubated with sera (diluted 1:100) for 1 h at RT. After washing, plates were incubated with recombinantly produced HRP-labelled antigen (CCHFV-NP, dilution 1:50.000) for 1 h at RT. After washing, TMB substrate was added and plates were incubated for 10 min at RT before stopping the reaction with H₂SO₄.

### IgM-Immune-Complex-ELISA

Plates (Nunc Maxisorp) were coated for at least 5 days at 4°C with 5 µg/ml His₁₀-3C-HsFcµR-Igl in PBS pH 7.4 supplemented with 0.01 % NaN₃ and phenol red (50 µl/well) in a humidified chamber. Wells were washed once with 0.2 ml Blocking Buffer (PBS pH 7.4 / 1% BSA) and were then incubated in 0.2 ml blocking buffer for 2 h at RT. Plates were washed three times with 0.3 ml Washing Buffer (100 mM Tris-base, 150 mM NaCl, 0.05 % Tween 20, pH 7.4) and incubated after application of the third application of Washing Buffer for 5 min at RT before aspiration. 25 µl of HRP-labelled recombinant antigen diluted 1:16.000 in Antigen Dilution Buffer (PBS pH 7.4, 1 % BSA, 0.5 % FCS, 1 % Nonidet P-40) were added to the wells and mixed with 25 µl of serum diluted 1:50 in Serum Dilution Buffer (PBS pH 7.4, 0.05 % ProClin 300 supplemented with phenol red) resulting in final dilutions of 1:32.000 for the HRP-labelled antigen and 1:100 for the sera. Plates were sealed and incubated for 24 h at 4°C in a humidified chamber. Plates were washed three times with 0.3 ml Washing Buffer and incubated after three applications of Washing Buffer at RT before aspiration. 50 µl TMB (KPL) per well were added and plates were incubated for 10 min at RT. After stopping the colorimetric reaction with 100 µl 1N H₂SO₄, optical density was read at 450/620 nm.

### Results

### The immunoglobulin-like domain of HsFcµR can be purified and refolded from the insoluble fraction of transformed E.coli pAPlacI^{Q}.

Two cDNA fragments encoding either the immunoglobulin-like domain of HsFcµR or the whole extracellular domain without signal peptide were ligated into a prokaryotic expression vector. Expression of the encoded fusion proteins His₁₀-3C-*Hs*FcµR-Igl and His₁₀-3C-*Hs*FcµR-ECD was induced in *E.coli* pAPlacI^{Q} transformed with the respective expression plasmids. The proteins were purified from the insoluble fraction of the bacterial lysate by Ni-NTA affinity chromatography under denaturing conditions and refolded by rapid dilution oxidative refolding. For His₁₀-3C-*Hs*FcµR-Igl, yields from 0.41 culture volume were 5.6 mg ± 0.1 mg protein after Ni-NTA-chromatography and 1.4 mg ± 0.1 mg Protein after refolding (n = 2). For His₁₀-3C-*Hs*FcµR-ECD, yields were 10.4 mg ± 0.4 mg after Ni-NTA-chromatography and 5.0 mg ± 0.7 mg Protein after refolding for His₁₀-3C-*Hs*FcµR-ECD (n = 2). While His₁₀-3C-*Hs*FcµR-Igl turned out to be stable under a variety of different buffer conditions, His₁₀-3C-*Hs*FcµR-ECD deteriorated quickly in standard buffers used for plate coating (data not shown). Thus, only His₁₀-3C-*Hs*FcµR-Igl was used for plate coating in further experiments.

### The immunoglobulin-like domain of HsFcµR can be used as a capture molecule for IgM/antigen immune complexes in IgM ELISA applications.

The potential of HsFcµR as a new capture molecule for IgM ELISA applications was tested according to the following protocol (for schematic overview of the test procedure see **Fig. 2**): Nunc Maxisorp plates plates were coated with His₁₀-3C-*Hs*FcµR-Igl. After blocking, serum samples were co-incubated over night at 4°C with HRP-labelled recombinant CCHFV antigen (CCHFV-NP^{HRP}). During this time period, immune complexes form and simultaneously bind to His₁₀-3C-*Hs*FcµR-Igl. After washing, bound IgM/CCHFV-NP^{HRP} immune complexes were detected by conversion of the HRP substrate TMB.

In total, 7 CCHF patient sera (pos 1 - 7) and 5 healthy blood donor sera (neg A - F) were analyzed. Anti-CCHFV IgM titers of patient sera were determined by immunofluorescence analysis, in addition, sera were tested using a commercially available CCHFV IgM ELISA (VectoCrimea-CHF-IgM µ-capture ELISA, VectorBest, Russia) (**Fig. 3A**)**.** Applying the IgM-ICB-ELISA protocol (schematic overview: **Fig. 2**), all seven IgM-positive sera were identified as positive using His₁₀-3C-*Hs*FcµR-Igl as capture molecule while the negative sera did not generate a signal (**Fig. 3B**)**.** Similarly, positive test results were obtained when anti-Lassa IgM positive patient sera were analyzed on His₁₀-3C-FcµR-Igl-coated plates (**Fig. 4**). Thus, His₁₀-3C-*Hs*FcµR-Igl is able to bind to IgM/antigen immune complexes.

It was further shown that at higher incubation temperature, incubation time can be shortened to several hours. Thus, the test also fulfills the time requirements for an acute diagnostics test.

### The immunoglobulin-like domain of HsFcµR displays very low affinity towards non-complexed IgM.

Next, we analyzed if His₁₀-3C-*Hs*FcµR-Igl, like anti-human IgM antibodies used in standard µ-capture IgM ELISA tests, also binds to non-complexed IgM molecules. Therefore, sera were applied to plates coated with either anti-human IgM (**Fig. 3C**) or His₁₀-3C-*Hs*FcµR-Igl (**Fig. 3D**) for 1 h at RT. Plates were washed thoroughly and then incubated with HRP-labeled recombinant antigen CCHFV-NP^{HRP} for 1 h at RT. After washing, bound HRP-labeled antigen was detected by TMB conversion. While on the anti-human IgM-coated plates, the CCHF IgM positive sera gave rise to clearly positive signals (**Fig. 3C**), no or only very weak signals were seen on the His₁₀-3C-*Hs*FcµR-Igl-coated plates (**Fig. 3D**). Thus, His₁₀-3C-*Hs*FcµR-Igl binds to non-complexed IgM molecules with a considerably lower affinity than anti-human IgM.

### The immunoglobulin-like domain of HsFcµR does not bind to IgG/antigen immune complexes.

To further characterize the binding specificity of His₁₀-3C-*Hs*FcµR-Igl, CCHFV-NP^{HRP} was co-incubated with a monoclonal anti-CCHFV-NP IgG antibody (Emmerich et al., 2010) on His₁₀-3C-*Hs*FcµR-Igl-coated plates. While the respective IgG/antigen immune complexes readily bound to plates coated with the IgG immune complex binding protein CD32 (data not shown), no signal was detected on His₁₀-3C-*Hs*FcµR-Igl-coated plates (**Fig. 5**)**.** Thus, His₁₀-3C-*Hs*FcµR-Igl does not bind to IgG/antigen immune complexes.

### The immunoglobulin-like domain of HsFcµR preferentially binds nascent IgM/antigen immune complexes.

To analyze if preformed IgM/antigen immune complexes can be bound by His₁₀-3C-*Hs*FcµR-Igl, CCHFV-NP^{HRP} and a anti-CCHFV-NP IgM positive serum were either co-incubated directly on the His₁₀-3C-*Hs*FcµR-Igl-coated plate for 24 h at 4°C or co-incubated for 19 h at 4°C in a test tube and then applied to a His₁₀-3C-*Hs*FcµR-Igl-coated plate for 5 h at 4°C. After washing, bound IgM/antigen complexes were quantified by TMB conversion (**Fig. 5**). A significantly higher signal was generated when IgM/antigen immune complexes were allowed to form directly on the His₁₀-3C-*Hs*FcµR-Igl-coated plate. Thus, His₁₀-3C-*Hs*FcµR-Igl preferentially binds nascent IgM/antigen complexes.

### Literature

Balmaseda A., Guzman, M.G.et al. (2003). "Diagnosis of Dengue Virus Infection by Detection of Specific Immunoglobulin M (IgM) and IgA Antibodies in Serum and Saliva" Clin Vaccine Immunol 10(2):317-322.
Berrow et al. (2007). "A versatile ligation-independent cloning method suitable for high-throughput expression screening applications". Nucleic Acids Res. Mar 2007; 35(6): e45.
Duermeyer W, Wielaard F. et al., (1979). "A new principle for the detection of specific IgM antibodies applied to an ELISA for hepatitis A". J Med Virol 4:25-32.
Emmerich et al. (2013). "Detection of Serotype-Specific Antibodies to the Four Dengue Viruses Using an Immune Complex Binding (ICB) ELISA." PLoS Negl Trop Dis. 2013 December; 7(12): e2580.
Hitoshi Y, Lorens J, Kitada SI, Fisher J, LaBarge M, Ring HZ, et al. Toso, a cell surface, specific regulator of Fas-induced apoptosis in T cells. Immunity. 1998 Apr;8(4):461-71.
Hoover RG, Dieckgraefe BK, Lynch RG. T cells with Fc receptors for IgA: induction of T alpha cells in vivo and in vitro by purified IgA. J Immunol. 1981 Oct;127(4):1560-3. Kubagawa H, Oka S, Kubagawa Y, Torii I, Takayama E, Kang DW, et al. Identity of the elusive IgM Fc receptor (FcmuR) in humans. The Journal of experimental medicine. 2009 Nov 23;206(12):2779-93.
Kubagawa H, Oka S, Kubagawa Y, Torii I, Takayama E, Kang DW, et al. The long elusive IgM Fc receptor, FcmuR. Journal of clinical immunology. 2014 Jul;34 Suppl 1:S35-45.
Maruoka T, Nagata T, Kasahara M. Identification of the rat IgA Fc receptor encoded in the leukocyte receptor complex. Immunogenetics. 2004 Jan;55(10):712-6.
Moretta L, Webb SR, Grossi CE, Lydyard PM, Cooper MD. Functional analysis of two human T-cell subpopulations: help and suppression of B-cell responses by T cells bearing receptors for IgM or IgG. The Journal of Experimental medicine. 1977 Jul 1;146(1):184-200.
Ohno T, Kubagawa H, Sanders SK, Cooper MD. Biochemical nature of an Fc mu receptor on human B-lineage cells. The Journal of experimental medicine. 1990 Oct 1;172(4):1165-75.
Ölschläger S, Gabriel M, Schmidt-Chanasit J, Meyer M, Osborn E, et al. (2011). Complete sequence and phylogenetic characterisation of Crimean-Congohemorrhagic fever virus from Afghanistan. J Clin Virol 50: 90-92.
Ong, L.Y., T. Pang, et al., (1989). "A simple adherence test for detection of IgM antibodies in typhoid". J Medical Microbiol 29(3):195-198.
Shibuya A, Sakamoto N, Shimizu Y, Shibuya K, Osawa M, Hiroyama T, et al. Fc alpha/mu receptor mediates endocytosis of IgM-coated microbes. Nat Immunol. 2000 Nov;1(5):441-6.
van der Boog PJ, van Zandbergen G, de Fijter JW, Klar-Mohamad N, van Seggelen A, Brandtzaeg P, et al. Fc alpha RI/CD89 circulates in human serum covalently linked to IgA in a polymeric state. J Immunol. 2002 Feb 1;168(3):1252-8.
Vasquez, Hafner et al. (2007). "Evaluation of immunoglobulin M and G capture enzyme-linked immunosorbent assay Panbio kits for diagnostic dengue infections". J Clin Virol. 39(3):194-8.
Vorndam, V., G. Kuno (1997). "Laboratory diagnosis of dengue virus infections", 313-333. In D. J. Gubler and G. Kuno (ed.), Dengue and dengue hemorrhagic fever. CAB International, New York, N.Y.
EP 2492689 A1

## Claims

1. Use of an FcµR immobilized on a solid support for *in vitro* quantifying antibodies in the form of immune complexes, wherein the immune complexes are formed by mixing an antigen with a sample comprising antibodies.

2. The use of claim 1, wherein the antigen is a detectable antigen such as a labelled antigen selected from the group comprising an enzyme labelled antigen and a biotin labelled antigen, or an antigen which comprises more than one epitope.

3. The use of any of the previous claims, wherein the addition of antigen to the sample is performed in the presence of FcµR.

4. The use of any of the previous claims, wherein the antibodies are derived from a biological sample.

5. The use of any of the previous claims, wherein the antibodies are derived from a species selected from the group comprising human, goat, sheep, horse, cattle, bird, such as chicken, and rodent, such as mouse, rat, rabbit, guinea pig and hamster.

6. The use of any of the previous claims, wherein the antibodies are IgM or IgA antibodies, preferably, IgM antibodies.

7. The use of any of the previous claims, wherein the the FcµR is Faim 3/Toso or a functional fragment thereof capable of binding to immune complexes or CD351 or a functional fragment thereof capable of binding to immune complexes.

8. The use of any of the previous claims, wherein the FcµR is a functional fragment of Faim 3/Toso capable of binding to immune complexes comprising or essentially consisting of the Ig-like domain of Faim 3/Toso, optionally, in the form of a fusion protein.

9. The use of any of claims 7 or 8, wherein Faim 3/Toso, CD351 or the functional fragment thereof comprises an amino acid sequence having at least 80% sequence identity with human Faim 3/Toso, CD351 or the respective fragment.

10. The use of any of the previous claims, wherein the FcµR comprises SEQ ID NO: 2.

11. A method for in vitro quantifying antibodies in the form of immune complexes, comprising
a) coating a solid support with an FcµR under conditions suitable for binding of the FcµR to the solid support,
b) incubating the solid support with a composition comprising an IgM or IgA antibody and an antigen, wherein said antibody and said antigen are capable of forming an immune complex, and wherein said immune complexes are formed by adding an antigen to a sample comprising antibodies, and
c) washing the solid support, and then
d) determining quantity of the immune complex.

12. The method of claim 11, wherein the antigen is a labelled antigen selected from the group comprising an enzyme labelled antigen and a biotin labelled antigen.

13. The method of any of claims 11 to 12, wherein the FcµR is Faim 3/Toso or a functional fragment thereof capable of binding to immune complexes or CD351 or a functional fragment thereof capable of binding to immune complexes.

14. A method for diagnosing an infection with a pathogen in a subject, wherein the method of any of claims 11 to 13 is carried out, wherein the composition comprising an antibody is a sample from the subject, and wherein the antigen which can be specifically bound by said antibody is an antigen derivable from the pathogen, such as an envelope protein of a virus.

15. A protein comprising essentially consisting of SEQ ID NO: 2 or essentially consisting of a protein having at least than 80% amino acid identity to SEQ ID NO: 2, optionally, linked to a Tag suitable for purification such as a His-Tag.
